(19) Europäisches Patentamt / European Patent Office / Office européen des brevets

(11) EP 2 518 050 A1

(12) EUROPEAN PATENT APPLICATION
published in accordance with Art. 153(4) EPC

(43) Date of publication:
**31.10.2012 Bulletin 2012/44**

(21) Application number: **10839402.4**

(22) Date of filing: **21.12.2010**

(51) Int Cl.:
***C07C 231/20*** (2006.01) ***C07C 231/18*** (2006.01)
***C07C 235/84*** (2006.01) ***C12P 41/00*** (2006.01)
***C07B 53/00*** (2006.01) ***C07B 57/00*** (2006.01)
***C07B 61/00*** (2006.01)

(86) International application number:
**PCT/JP2010/073013**

(87) International publication number:
**WO 2011/078172 (30.06.2011 Gazette 2011/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.12.2009 JP 2009295474**

(71) Applicant: **Kaneka Corporation**
**Osaka-shi, Osaka 530-8288 (JP)**

(72) Inventors:
- **UEKITA, Ken**
  **Takasago-shi**
  **Hyogo 676-8688 (JP)**
- **NISHIYAMA, Akira**
  **Takasago-shi**
  **Hyogo 676-8688 (JP)**
- **TAOKA, Naoaki**
  **Takasago-shi**
  **Hyogo 676-8688 (JP)**

(74) Representative: **HOFFMANN EITLE**
**Patent- und Rechtsanwälte**
**Arabellastrasse 4**
**81925 München (DE)**

# (54) PROCESS FOR PRODUCTION OF OPTICALLY ACTIVE 3-SUBSTITUTED GLUTARIC ACID MONOAMIDE

(57) The process for producing an optically active 3-substituted glutaric acid monoamide is characterized in comprising the step of precipitating the optically active 3-substituted glutaric acid monoamide by mixing an acid and a mixed liquid containing an optically active 3-substituted glutaric acid monoamide represented by the following formula (2):

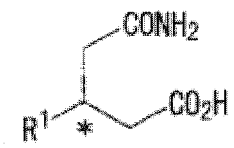

(2)

wherein * indicates an asymmetric carbon atom; $R^1$ is a $C_{1-8}$ alkyl group, a $C_{2-8}$ alkenyl group, a $C_{2-8}$ alkynyl group, a $C_{4-20}$ aryl group or a $C_{5-20}$ aralkyl group; and the alkyl group, the alkenyl group, the alkynyl group, the aryl group and the aralkyl group may have a substituent, a basic compound, water and an organic solvent.

EP 2 518 050 A1

## Description

TECHNICAL FIELD

The present invention relates to a process for producing an optically active 3-substituted glutaric acid monoamide having high optical purity, which is useful as an intermediate for a drug.

BACKGROUND OF THE INVENTION

As a process for producing an optically active 3-substituted glutaric acid monoamide, for example, the following processes are known:

· optical resolution process, in which a racemate of a 3-substituted glutaric acid monoamide is treated with optically active methylbenzylamine, optically active phenylethylamine or the like to form a diastereomeric salt, and the racemate is optically-resolved by fractional crystallization (Patent Document 1, Non-patent document 1 and the like); and
· nitrile hydratase process, in which one nitrile group of a 3-substituted glutardinitrile is asymmetrically hydrolyzed using nitrile hydratase, 1) the obtained mononitrilemonocarboxylic acid is subjected to Curtius transfer reaction and then hydrolyzed using an acid, or 2) the nitrile group of the obtained mononitrilemonocarboxylic acid is hydrolyze and then subjected to Hoffmann transfer reaction (Non-patent document 2).

PRIOR ART

PATENT DOCUMENT

Patent document 1: WO97/22578

NON-PATENT DOCUMENT

Non-patent document 1: Journal of the Chemical Society, Perkin Transactions, 2, 1997(4), 763-768
Non-patent document 2: Tetrahedron Asymmetry 2001, 12, 3367-3373

SUMMARY OF THE INVENTION

PROBLEM TO BE SOLVED BY THE INVENTION

However, according to the optical resolution process, a desired optical substance cannot be theoretically produced with more than 50% yield, since a racemate should be optically resolved. In addition, the optical resolution process is complicated as a means for improving optical purity, since an optically active amine should be used as an optical resolution agent.
On the other hand, it is not necessary in the nitrile hydratase process to waste the unwanted optical isomer. However, the yield of the asymmetrical hydrolyzation is about 67%, the yield of Curtius transfer reaction is 43% and the yield of the hydrolyzation using an acid is 78%. Therefore, the total yield of the three steps is low as 22%. In addition, when the hydrolyzation using an acid and Hoffmann transfer reaction are carried out after the asymmetrical hydrolyzation, the yields are respectively 79% and 71%. Therefore, total yield of the three steps is also low as about 38%. Further, the stereoselectivity of the process should be improved. Furthermore, the target compound has to be purified for the use as an intermediate for a drug.
As described above, conventional processes to be presently known for producing an optically active 3-substituted glutaric acid monoamide are not advantageous to an industrial production in terms of yield and product quality.
In addition, it is not known how to easily improve the optical purity of an optically active 3-substituted glutaric acid monoamide. For example, in the case that an optically active 3-substituted glutaric acid monoamide was dissolved in a basic aqueous solution and then the 3-substituted glutaric acid monoamide was precipitated by adding an acid, the optical purity thereof could not be improved at all. Therefore, it is strongly desired to easily improve the optical purity of an optically active 3-substituted glutaric acid monoamide.
An objective of the present invention is to provide a process for producing an optically active 3-substituted

glutaric acid monoamide, by which process the optical purity of an optically active 3-substituted glutaric acid monoamide can be easily improved.
Another objective of the present invention is to provide a process for producing an optically active 3-substituted glutaric acid monoamide having high optical purity, which is useful as an intermediate for a drug, with high yield.

MEANS TO SOLVE THE PROBLEM

Hereinafter, the present invention is described.

[1] A process for producing an optically active 3-substituted glutaric acid monoamide, comprising the step of precipitating the optically active 3-substituted glutaric acid monoamide by mixing an acid and a mixed liquid containing an optically active 3-substituted glutaric acid monoamide represented by the following formula (2):

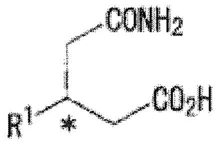
(2)

wherein * indicates an asymmetric carbon atom; $R^1$ is a $C_{1-8}$ alkyl group, a $C_{2-8}$ alkenyl group, a $C_{2-8}$ alkynyl group, a $C_{4-20}$ aryl group or a $C_{5-20}$ aralkyl group; and the alkyl group, the alkenyl group, the alkynyl group, the aryl group and the aralkyl group may have a substituent,
a basic compound, water and an organic solvent.

[2] The process according to [1], wherein $R^1$ is an n-propyl group or a 4-chlorophenyl group.

[3] The process according to [1] or [2], wherein the organic solvent is at least one selected from the group consisting of methanol, ethanol, isopropanol, tetrahydrofuran, acetonitrile and acetone.

[4] The process according to any one of [1] to [3], wherein the amount of the organic solvent is not less than 0.5 parts by mass and not more than 50 parts by mass relative to 1 part by mass of the optically active 3-substituted glutaric acid monoamide.

[5] The process according to any one of [1] to [4], wherein the basic compound is at least one selected from the group consisting of an alkali metal salt, ammonia and triethylamine.

[6] The process according to any one of [1] to [5], wherein the pH of the mixed liquid is not less than 6.

[7] A process for producing an optically active 3-substituted glutaric acid monoamide, comprising the steps of:
reacting a 3-substituted glutaric anhydride represented by the following formula (3):

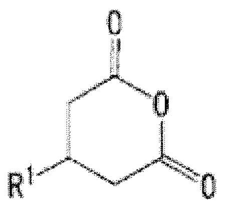
(3)

wherein $R^1$ is a $C_{1-8}$ alkyl group, a $C_{2-8}$ alkenyl group, a $C_{2-8}$ alkynyl group, a $C_{4-20}$ aryl group or a $C_{5-20}$ aralkyl group; and the alkyl group, the alkenyl group, the alkynyl group, the aryl group and the aralkyl group may have a substituent,
with an alcohol in the presence of an asymmetric organocatalyst, to obtain an optically active 3-substituted glutaric acid monoester represented by the following formula (4):

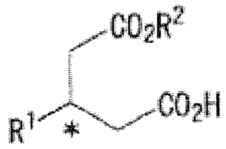

(4)

wherein * indicates an asymmetric carbon atom; $R^1$ is the same as the above; $R^2$ corresponds to a residue obtained by removing the OH group from the alcohol, and is an optionally substituted $C_{1-8}$ alkyl group, an optionally substituted $C_{2-8}$ alkenyl group or an optionally substituted $C_{7-15}$ aralkyl group; and
then carrying out an aminolysis reaction of the optically active 3-substituted glutaric acid monoester (4);
wherein the optically active 3-substituted glutaric acid monoamide is represented by the following formula (2):

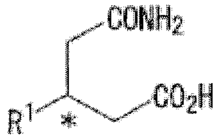

(2)

wherein $R^1$ and * are the same as the above.

[8] The process according to [7], wherein the asymmetric organocatalyst is an optically active tertiary amine compound.

[9] The process according to [7], wherein the asymmetric organocatalyst is a compound represented by the following formula (5) or the following formula (6):

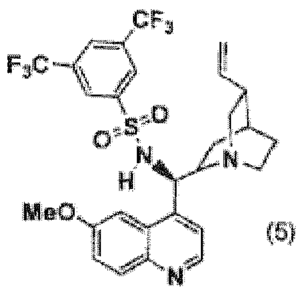

(5)

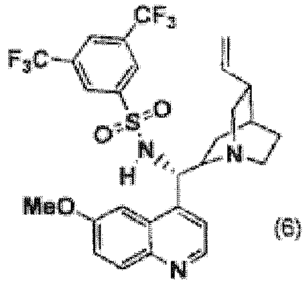

(6)

.

[10] The process according to any one of [7] to [9], wherein the alcohol is methanol, ethanol or benzyl alcohol.

[11] A process for producing an optically active 3-substituted glutaric acid monoamide, comprising the steps of:
hydrolyzing a 3-substituted glutaric acid diester represented by the following formula (7):

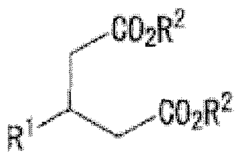

(7)

wherein $R^1$ is a $C_{1-8}$ alkyl group, a $C_{2-8}$ alkenyl group, a $C_{2-8}$ alkynyl group, a $C_{4-20}$ aryl group or a $C_{5-20}$ aralkyl group; the alkyl group, the alkenyl group, the alkynyl group, the aryl group and the aralkyl group may have a substituent; $R^2$ is an optionally substituted $C_{1-8}$ alkyl group, an optionally substituted $C_{2-8}$ alkenyl group or an optionally substituted $C_{7-15}$ aralkyl group,
by using an enzyme having an asymmetric hydrolysis activity, to obtain an optically active 3-substituted glutaric acid monoester represented by the following formula (4):

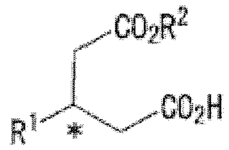
(4)

wherein * indicates an asymmetric carbon atom; and $R^1$ and $R^2$ are the same as the above, and then carrying out an aminolysis reaction of the optically active 3-substituted glutaric acid monoester (4); wherein the optically active 3-substituted glutaric acid monoamide is represented by the following formula (2):

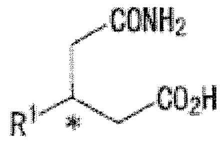
(2)

wherein $R^1$ and * are the same as the above.

[12] The process according to [11], wherein the enzyme having the asymmetric hydrolysis activity is an immobilized lipase.

[13] The process according to [11], wherein the enzyme having the asymmetric hydrolysis is Novozyme 435.

[14] The process according to any one of [11] to [13], wherein $R^2$ is a methyl group or an ethyl group.

[15] A process for producing an optically active 3-substituted glutaric acid monoamide, comprising the steps of: mixing the optically active 3-substituted glutaric acid monoamide obtained by the process according to any one of [7] to

[14] with a basic compound, water and an organic solvent to obtain a solution; and then further mixing the solution with an acid, to precipitate the optically active 3-substituted glutaric acid monoamide.

EFFECT OF THE INVENTION

According to the present invention, it is possible to easily improve the optical purity of an optically active 3-substituted glutaric acid monoamide (2) by only mixing an acid and a mixed liquid containing the monoamide (2), a basic compound and an organic solvent for precipitation.
In addition, according to the present invention, it is possible to produce an optically active 3-substituted glutaric acid monoamide (2) having high optical purity with high yield by reacting a 3-substituted glutaric anhydride (3) with an alcohol in the presence of an asymmetric organocatalyst or treating a 3-substituted glutaric acid diester (7) with a prescribed enzyme to produce a 3-substituted glutaric acid monoester (4), and then carrying out an aminolysis reaction of the monoester (4).

DETAILED DESCRIPTION OF THE INVENTION

As a process for producing an optically active 3-substituted glutaric acid monoamide, the following three routes are exemplified.

1. First route

1-1. Asymmetric hydrolysis

In the first route, a 3-substituted glutaric acid diamide (1) represented by the following formula (1):

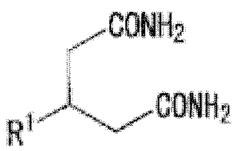
(1)

is treated with an enzyme having an asymmetric hydrolysis activity, to obtain an optically active 3-substituted glutaric acid monoamide (2) represented by the following formula (2):

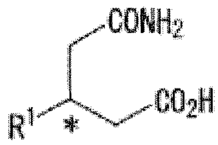

(2)

.

Hereinafter, the above enzyme is referred to as "amidase type enzyme".

In the above-described formulae (1) and (2), $R^1$ is a $C_{1-8}$ alkyl group, a $C_{2-8}$ alkenyl group, a $C_{2-8}$ alkynyl group, a $C_{4-20}$ aryl group or a $C_{5-20}$ aralkyl group. The alkyl group may be linear or branched. The alkyl group is exemplified by a methyl group, an ethyl group, an n-propyl group, an isopropyl group, an n-butyl group, an isobutyl group, a tert-butyl group, a pentanyl group, a hexanyl group, a heptanyl group, an octanyl group and the like. The alkenyl group is exemplified by an ethenyl group, a propenyl group, a butenyl group, a pentenyl group, a hexenyl group, a heptenyl group, an octenyl group and the like. The alkynyl group is exemplified by an ethynyl group, a propynyl group, a butynyl group, a pentynyl group, a hexynyl group, a heptynyl group, an octynyl group and the like. The aryl group is defined as a group having an aromatic ring, and the aromatic ring may be an aromatic heterocyclic ring in addition to a hydrocarbon aromatic ring. The aryl group is exemplified by a phenyl group, a naphthyl group, an anthranil group, a pyridyl group, a pyrimidyl group, an indanyl group, an indenyl group and the like, and is preferably an aromatic ring itself. The aralkyl group is defined as an alkyl group bound by an aromatic ring, and may be an alkyl group bound by an aromatic heterocyclic ring in addition to an alkyl group bound by an aromatic hydrocarbon ring. The aralkyl group is exemplified by a benzyl group, a naphthylmethyl group, an anthranilmethyl group, a pyridylmethyl group, a pyrimidylmethyl group, an indanylmethyl group, an indenylmethyl group and the like, and is preferably an alkyl group bound by an aromatic hydrocarbon ring.

The alkyl group, the alkenyl group, the alkynyl group, the aryl group and the aralkyl group may have one or more substituents, particularly substituents other than a hydrocarbon group. Such a substituent is exemplified by a halogen atom, a hydroxy group, an amino group, a nitro group and the like. A halogen atom is exemplified by a fluorine atom, a chlorine atom, a bromine atom and the like, and is preferably a chlorine atom.

The $R^1$ is preferably an n-propyl group, an isobutyl group, a phenyl group or a 4-chlorophenyl group, more preferably an n-propyl group or a 4-chlorophenyl group, and particularly preferably a 4-chlorophenyl group.

The optical purity of the above-described compound (1) (diamide (1)) is generally low, and the compound (1) is usually a racemate. The optical purity of the above-described compound (2) (monoamide (2)) is high. The "*" in the formula (2) indicates an asymmetric carbon atom, and an absolute configuration may be R or S.

As described above, the compound (1) (diamide (1)) is treated with an amidase type enzyme. In the present invention, the "enzyme" may be isolated from a microorganism and also contains not only a microorganism which can produce an enzyme protein but also a transformed microorganism transgenic for a gene coding an enzyme protein. In addition, the above-described microorganism and transformed microorganism may be immobilized, disrupted or dried, and such processed microorganisms are included in the term "enzyme" as long as the microorganisms have a desired enzymatic activity.

The term "microorganism" may be a culture solution containing a fungus body and a dispersion containing a fungus body in addition to a fungus body itself.

The term "processed microorganism" is exemplified by an extract, a freeze-dried fungus body, an acetone-dried fungus body and a fragment of the fungus bodies. In addition, the term "processed microorganism" may be an enzyme itself having the desired asymmetric hydrolysis activity and a partly-purified enzyme. The enzyme and fungus body may be immobilized to be used by a conventional means. Such an immobilization may be carried out by a conventional method known to the person skilled in the art, such as a crosslinking method, a physical adsorption method and a packaging method.

All of the microorganisms described later in the specification are available from a depository authority and various research institutions, and may be obtained from nature. For example, a microorganism identified by NBRC number is available from National Institute of Technology and Evaluation, a microorganism identified by FERM number is available from The National Institute of Advanced Industrial Science and Technology, a microorganism identified by IAM number is available from Center for Bioinformatics in Institute of Molecular and Cellular Biosciences, the University of Tokyo, a microorganism identified by JCM number is available from Microbe Division / Japan Collection of Microorganisms RIKEN BioResource Center.

The desired microorganism may be obtained from nature by enrichment culture using a raw material compound for an enzyme reaction or an agent for inducing enzyme production. In the first route, a 3-substituted glutaric acid diamide (1) is used as a raw material compound for an enzyme reaction.

A microorganism having amidase type enzyme is not particularly limited, and exemplified by a microorganism

selected from the group consisting of genus Achromobacter, genus Alcaligenes, genus Arthrobacter, genus Acinetobacter, genus Cellulomonas, genus Delftia, genus Nocardia, genus Pseudomonas, genus Rhodococcus and genus Stenotrophomonas.

A microorganism having the ability to produce a 3-substituted glutaric acid monoamide (2) of which absolute configuration is S in the above-described microorganisms is exemplified by a microorganism selected from the group consisting of genus Achromobacter, genus Alcaligenes, genus Arthrobacter, genus Acinetobacter, genus Cellulomonas, genus Delftia, genus Pseudomonas and genus Stenotrophomonas. Such a microorganism is preferably Achromobacter sp., Achromobacter xylosoxidans subsp. denitrificans, Alcaligenes xylosoxidans subsp. denitrificans, Arthrobacter sp., Acinetobacter sp., Cellulomonas fimi, Delftia acidovorans, Pseudomonas sp. or Stenotrophomonas sp. The microorganism is more preferably Achromobacter xylosoxidans subsp. denitrificans IFO15125, Achromobacter xylosoxidans subsp. denitrificans IFO12669, Cellulomonas fimi IFO15513, Delftia NBRC13582 or Delftia NBRC14950.

A microorganism having the ability to produce a 3-substituted glutaric acid monoamide (2) of which absolute configuration is R in the above-described microorganisms is exemplified by a microorganism selected from the group consisting of genus Alcaligenes, genus Acinetobacter, genus Nocardia and Rhodococcus. Such a microorganism is preferably Alcaligenes sp., Acinetobacter sp., Nocardia globerula, Rhodococcus erythropolis and Rhodococcus rhodochrous. The microorganism is more preferably Alcaligenes sp. IFO14130, Rhodococcus erythropolis IFO12538, Rhodococcus erythropolis IFO12539, Rhodococcus erythropolis IAM1440, Rhodococcus erythropolis IAM1452, Rhodococcus erythropolis IAM1474, Rhodococcus erythropolis IAM12122 or Rhodococcus rhodochrous NBRC15564.

The microorganism may be wild strain or mutant strain. Alternatively, a microorganism induced by a genetic method such as cell fusion and gene manipulation can be also used. For example, a genetically-modified microorganism which can produce the present invention enzyme can be obtained by a process described in WO98/35025 containing the steps of isolating and/or purifying the enzyme and determining the amino acid sequence of the enzyme partially or entirely, preparing the DNA sequence encoding the enzyme on the basis of the amino acid sequence information, inserting the DNA into other microorganism to obtain a living modified microorganism, and culturing the living modified microorganism to obtain the enzyme.

The living modified microorganism is exemplified a transgenic microorganism transformed with a plasmid having a DNA encoding the hydrolytic enzyme. As a host microorganism, Escherichia coli is preferred.

A culture medium for culturing a microorganism is not limited as long as the microorganism can grow. For example, a general liquid culture medium containing a carbohydrate such as glucose and sucrose as a carbon source; an alcohol such as ethanol and glycerol; a fatty acid, such as oleic acid and stearic acid, and an ester thereof; an oil such as canola oil and soybean oil; a nitrogen source such as ammonium sulfate, sodium nitrate, peptone, casamino acid, corn steep liquor, bran and yeast extract; a mineral salt such as magnesium sulfate, sodium chloride, calcium carbonate, potassium hydrogenphosphate and potassium dihydrogenphosphate; and other nutrient source such as malt extract and meat extract can be used.

In order to promote the production of the enzyme by a microorganism, a 3-substituted glutaric acid diamide (1) and an inducing agent may be added to a culture medium. Such an inducing agent is exemplified by a nitrile, a lactam compound, an amide and the like. Such a nitrile is exemplified by acetonitrile, isovaleronitrile, propionitrile, pivalonitrile, n-butyronitrile, isobutyronitrile, n-capronitrile, 3-pentenenitrile and the like. Such a lactam compound is exemplified by γ-butyrolactam, 5-valerolactam, ε-caprolactam and the like. Such an amide is exemplified by crotonamide, benzamide, propionamide, acetamide, n-butyramide, isobutyramide, n-valeramide, n-capronamide, methacrylamide, phenylacetamide, cyclohexanecarboxamide and the like.

The amount of an inducing agent to be added in a culture medium is not less than 0.05 mass% and preferably not less than 0.1 mass%, and not more than 2.0 mass% and preferably not more than 1. mass%. A microorganism is cultured in an aerobic condition, and generally a culture time is not less than about 1 day and not more than about 5 days, the pH of a culture medium is not less than 3 and not more than 9, and a culture temperature is not less than 10°C and not more than 50°C.

In the present invention, for example, a stereoselective hydrolysis reaction of a 3-substituted glutaric acid diamide (compound (1)) can be carried out by adding a 3-substituted glutaric acid diamide (1) as a raw compound and the above-described fungus body or the processed fungus body into an appropriate solvent and stirring the mixture with adjusting the pH.

A reaction condition differs depending on an enzyme to be used, microorganism or processed microorganism, a substance concentration and others. In general, a substrate concentration is not less than 0.1 mass% and preferably not less than 1 mass%, and not more than 99 mass% and preferably not more than 60 mass%. For example, a reaction temperature is not less than 10°C and preferably not less than 20°C and more preferably not less than 30°C, and not more than 70°C and preferably not more than 60°C and more preferably not more than 50°C. For example, the pH for the reaction is not less than 4 and preferably not less than 6, and not more than 11 and preferably not more than 9. A reaction time is not less than 1 hour and not more than 120 hours, and preferably not less than 1 hour and not more than 72 hours. A substrate may be added at a time or in a continuous manner. The reaction may be carried out in a

batch manner or a continuous manner.

An optically active 3-substituted glutaric acid monoamide (2) produced by the reaction can be isolated by a conventional method. Specifically, for example, the optically active 3-substituted glutaric acid monoamide (2) produced by the hydrolysis reaction is extracted from the reaction mixture containing the monoamide (2) using an organic solvent such as ethyl acetate and toluene, and the organic solvent is distilled away from the extract to obtain the target compound. Alternatively, the filtrate obtained by removing a microorganism body from the reaction mixture is neutralized by adding an acid such as hydrochloric acid and sulfuric acid thereto to precipitate the target compound as a solid, and the target compound is filtered for isolation.

The thus obtained optically active 3-substituted glutaric acid monoamide represented by the formula (2) (monoamide (2)) has high purity sufficient for the use in the subsequent step. However, the optical purity cannot be improved according to the above isolation method. Therefore, when the stereoselectivity of the hydrolysis reaction is not sufficient, the optical purity of the obtained target compound has to be further improved for the use as a raw material compound of an intermediate compound for a drug. For example, when the amidase type enzyme is used, the optical purity of the monoamide (2) may be exemplified by not less than 60%ee and preferably not less than 65%ee, and not less than 95%ee in some cases, but may be less than 90%ee and particularly not more than 85%ee in some cases.

1-2. Means for improving optical purity

The present inventors developed a process by which the optical purity of the optically active monoamide (2) can be easily improved. The process can be used for improving the optical purity of the optically active monoamide (2) which is obtained by various methods, particularly can be used for improving the optical purity of the optically active monoamide (2) which is obtained by the above-described first route. In the process for improving the optical purity developed by the present inventors, a mixed liquid containing the optically active monoamide (2), a basic compound, water and an organic solvent is mixed with an acid to precipitate the optically active monoamide (2) as a solid.

In the present invention, the term "solid" means a crystal, an amorphous or a mixture thereof.

The basic compound to be used can form a salt with a carboxylic acid. The basic compound is exemplified by an alkali metal salt such as sodium hydroxide, potassium hydroxide, sodium carbonate, potassium carbonate and sodium hydrogencarbonate; ammonia; triethylamine; and the like. The basic compound is preferably sodium hydroxide, potassium hydroxide or ammonia. One of the basic compound may be used alone, and two or more basic compounds may be used in combination.

The amount of the basic compound is adjusted so that the mixture becomes neutral or basic. Specifically, the basic compound is added so that the pH becomes not less than 6, preferably not less than 8, more preferably not less than 11, and particularly preferably not less than 13.

The amount of water is adjusted so that the salt of monoamide (2) and the basic compound can be dissolved. For example, the amount of water relative to 1 part by mass of the monoamide (2) may be not less than 0.5 parts by mass, preferably not less than 1 part by mass, more preferably not less than 3 parts by mass, and not more than 50 parts by mass, preferably not more than 30 parts by mass, and more preferably not more than 10 parts by mass.

The organic solvent is exemplified by an alcohol solvent such as methanol, ethanol, n-propanol, isopropanol, n-butanol, tert-butanol and ethylene glycol; an ester solvent such as ethyl acetate, n-propyl acetate and isopropyl acetate; an ether solvent such as tetrahydrofuran, diethylether, 1,4-dioxane, methyl tert-butyl ether, diisopropylether, cyclopentyl methyl ether and ethylene glycol dimethyl ether; a ketone solvent such as acetone and methyl ethyl ketone; a nitrile solvent such as acetonitrile and propionitrile; an aromatic hydrocarbon solvent such as benzene, toluene and xylene; an aliphatic hydrocarbon solvent such as pentane, hexane, heptane and methylcyclohexane; a halogenated solvent such as methylene chloride, 1,2-dichloroethane and chlorobenzene; a sulfoxide solvent such as dimethylsulfoxide; an amide solvent such as N,N-dimethylformamide, N,N-dimethylacetamide, N,N-diethylacetamide, N-methyl-2-pyrrolidone, N-ethyl-2-pyrrolidone, N-methyl-ε-caprolactam and hexamethylphosphoramide; a urea solvent such as dimethylpropyleneurea; a phosphoric triamide solvent such as hexamethylphosphoric triamide. One of the solvent may be used alone, and two or more solvents may be used in combination. When two or more solvents are used in combination, the mixing ratio is not particularly limited.

As the organic solvent, a water-miscible organic solvent is preferred. Specifically, an alcohol solvent such as methanol, ethanol, n-propanol, isopropanol, n-butanol, tert-butanol and ethylene glycol; an ether solvent such as tetrahydrofuran, diethyl ether, 1,4-dioxane, methyl tert-butyl ether, diisopropylether and ethylene glycol dimethyl ether; a ketone solvent such as acetone and methyl ethyl ketone; a nitrile solvent such as acetonitrile and propionitrile are preferred. The organic solvent is more preferably methanol, ethanol, isopropanol, tetrahydrofuran, acetonitrile or acetone.

The amount of the organic solvent to be used relative to 1 part by mass of the monoamide (2) is exemplified by not less than 0.5 parts by mass, preferably not less than 1 part by mass, and more preferably not less than 3 parts by mass. On the other hand, too much organic solvent is not preferred in terms of cost and lower yield. The amount thereof relative to 1 part by mass of the monoamide (2) is therefore exemplified by not more than 50 parts by mass,

preferably not more than 30 parts by mass, and more preferably not more than 20 parts by mass.

The amount of the organic solvent relative to 100 parts by mass of water is exemplified by not less than 10 parts by mass, preferably not less than 20 parts by mass, more preferably not less than 30 parts by mass, and not more than 500 parts by mass, preferably not more than 300 parts by mass, and more preferably not more than 200 parts by mass.

The order of mixing the monoamide (2), the basic compound, water and the organic solvent is not particularly limited.

In order to precipitate a solid, after the monoamide (2), the basic compound, water and the organic solvent are mixed, the mixture is mixed with an acid. In general, a solid can be precipitated by the procedure only.

However, a solid may not be possibly precipitated by mixing an acid only in some cases depending on the kind or amount of the basic compound, the kind of the organic solvent and others. In such a case, a solid may be precipitated by further various procedures in addition to the above-described fundamental procedure. Such further procedures correspond to the specific embodiments of the above-described fundamental procedure, and are exemplified by the following methods:

(a) a method in which after the mixed liquid of the monoamide (2), the basic compound, water and the organic solvent is mixed with an acid, a poor solvent is further added thereto to precipitate a solid;
(b) a method in which after the mixed liquid of the monoamide (2) , the basic compound, water and the organic solvent is mixed with an acid, the obtained mixture is concentrated to precipitate a solid;
(c) a method in which after the mixed liquid of the monoamide (2), the basic compound, water and the organic solvent is mixed with an acid, the obtained mixture is cooled to precipitate a solid;
(d) a method in which after the mixed liquid of the monoamide (2), the basic compound, water and the organic solvent is mixed with an acid, the obtained mixture is concentrated and the solvent is substituted by a poor solvent to precipitate a solid;
(e) a method in which the addition of a poor solvent, concentration, cooling and substitution by a poor solvent exemplified in the above (a) to (d) are appropriately combined.

Regardless of the above-described methods, it is preferred that the monoamide (2) , the basic compound, water and the organic solvent are mixed, and the mixture is only mixed with an acid without carrying out the above-described methods (a) to (e) for obtaining a compound with high optical purity.

When a solid is precipitated, a seed solid may be added. A poor solvent used in the above methods (a) and (d) is exemplified by a hydrocarbon solvent such as toluene and hexane, water and the like.

The temperature for precipitating a solid is not particularly limited, and may be appropriately adjusted. For decreasing the production of impurity, the temperature is preferably not more than 100°C, more preferably not more than 80°C, and particularly preferably not more than 50°C. Also, the temperature is exemplified by not less than -20°C, preferably not less than -10°C, and more preferably not less than 0°C.

The acid is specifically exemplified by an inorganic acid such as hydrofluoric acid, hydrochloric acid, hydrobromic acid, hydroiodic acid, sulfuric acid, nitric acid, phosphoric acid and boric acid; a carboxylic acid such as formic acid, acetic acid, propionic acid, butyric acid, pivalic acid, chloroacetic acid, trichloroacetic acid, trifluoroacetic acid, benzoic acid and oxalic acid; a sulfonic acid such as methanesulfonic acid, trifluoromethanesulfonic acid, benzenesulfonic acid and p-toluenesulfonic acid; and the like. The acid is preferably hydrochloric acid, hydrobromic acid, sulfuric acid, acetic acid, oxalic acid, benzoic acid, methanesulfonic acid or p-toluenesulfonic acid, more preferably hydrochloric acid, hydrobromic acid, sulfuric acid, oxalic acid or methanesulfonic acid, and particularly preferably hydrochloric acid.

It is preferred that the acid is directly added, or preferably dissolved in water and the solution is added to the mixed liquid of the monoamide (2), the basic compound, water and the organic solvent. When an aqueous solution of the acid is added, the concentration of the acid is exemplified by not less than 10 mass%, preferably not less than 20 mass%, and more preferably not less than 30 mass%. The upper limit of the acid concentration is not limited, and the acid concentration is exemplified by not more than 60 mass%, preferably not more than 50 mass%, and more preferably not more than 40 mass%.

The monoamide (2) precipitated by the above procedure can be separated to be obtained by filtration under reduced pressure, filtration under increased pressure, centrifugation and others. When the optical purity of the solid is decreased due to residual mother liquid in the obtained solid, the solid may be washed with water or a mixed solvent of an organic solvent and water as necessary to improve quality.

The drying method of the solid is not particularly limited, and it is preferred that the solid is dried at not more than about 60°C under reduced pressure or in vacuo to avoid heat degradation and meltdown.

When the optical purity of the monoamide (2) should be further improved, the monoamide (2) is precipitated as the above again as necessary. Alternatively, the monoamide (2) may be precipitated by the following methods again:

(f) a method in which after the monoamide (2) is mixed with an organic solvent, water is further added thereto to

precipitate a solid;

(g) a method in which after the monoamide (2) is mixed with an organic solvent and water, a poor solvent is further added thereto to precipitate a solid;

(h) a method in which after the monoamide (2) is mixed with an organic solvent and water, the mixture is concentrated to precipitate a solid;

(i) a method in which after the monoamide (2) is mixed with an organic solvent and water, the mixture is cooled to precipitate a solid;

(j) a method in which after the monoamide (2) is mixed with an organic solvent and water, the mixture is concentrated and the solvent is substituted by a poor solvent to precipitate a solid.

The above methods (f) to (j) may be appropriately combined to precipitate a solid. When a solid is precipitated, a seed solid may be added. The organic solvent used in the methods (f) to (j) is exemplified by the above-described organic solvent. The poor solvent used in the methods (g) and (j) is exemplified by a hydrocarbon solvent such as toluene and hexane.

The optical purity of the thus obtained monoamide (2) is generally not less than 95%ee. It is possible by the present invention to obtain the monoamide (2) with not less than 98%ee, particularly not less than 99%ee generally-necessary for a drug.

For example, the diamide (1) can be produced by diamidating the after-described compound (7) (diester (7)). The diester (7) can be produced by diesterizing a 3-substituted glutaric acid represented by the following formula (9):

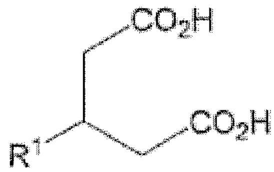

(9)

wherein $R^1$ is the same as the above.

In addition, an aminocarboxylic acid compound represented by the following formula (10):

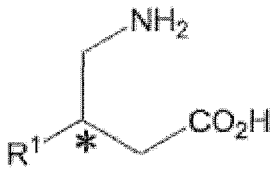

(10)

wherein $R^1$ and * are the same as the above,
can be produced by Hofmann rearrangement of the monoamide (2) obtained as the above.
In the case that the monoamide (2) is
3-(4-chlorophenyl)glutaric acid monoamide, the obtained aminocarboxylic acid is 4-amino-3-(4-chlorophenyl)butanoic acid, which is referred to as baclofen and useful as a drug having central muscle relaxant effect, analgetic effect and other effects.

2. Second route

In the second route, the monoamide (2) is produced by reacting, particularly adding, an alcohol to a 3-substituted glutaric anhydride represented by following formula (3):

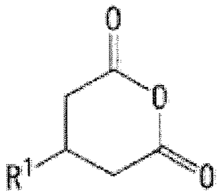

(3)

in the presence of an asymmetric organocatalyst, to obtain an optically active 3-substituted glutaric acid monoester (4):

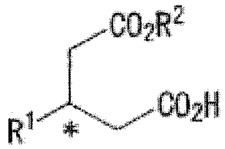

(4)

and then carrying out aminolysis reaction of the monoester (4).

In the formulae (3) and (4), $R^1$ is the same as the above. The optical purity of the compound (3) (acid anhydride (3)) is generally low, and the compound (3) is generally a racemate. The compound (4) (monoester (4)) has high optical purity. The "*" indicates an asymmetric carbon atom, and the absolute configuration may be R or S.

The $R^2$ is a $C_{1-8}$ alkyl group, a $C_{2-8}$ alkenyl group or a $C_{7-15}$ aralkyl group. The alkyl group may be linear or branched. The alkyl group is exemplified by a methyl group, an ethyl group, an n-propyl group, an isopropyl group, an n-butyl group, an isobutyl group and the like. The alkenyl group is exemplified by a propenyl group, a butenyl group and the like. The aralkyl group is exemplified by a benzyl group, a 1-phenethyl group and the like. The alkyl group, alkenyl group and aralkyl group may have one or two or more substituents, particularly a substituent other than a hydrocarbon group. The substituent is exemplified by a halogen atom, a hydroxy group, an amino group, a nitro group and the like.

The $R^2$ is preferably a methyl group, an ethyl group or a benzyl group, more preferably a methyl group or an ethyl group, and particularly preferably a methyl group.

When the monoester (4) is produced by reacting the acid anhydride (3) with an alcohol, $R^2$ corresponds to a residue obtained by removing OH from the used alcohol.

The asymmetric organocatalyst is not particularly limited, and specifically exemplified by a 1,2-diphenyl-1,2-ethanediamine derivative described in JP2007-119452A, such as optically active N,N-dimethyl-N'-3,5-bis(trifluoromethyl) benzenesulfonyl-1,2-di phenyl-1,2-ethanediamine and optically active N,N-dimethyl-N'-p-toluenesulfonyl-1,2-diphenyl-1,2-ethanediami ne; an optically active tetramisole derivative described in Organic Letters, 2006, 8(7), 1351-1354; a cinchona alkaloid derivative described in J.Am.Chem.Soc., 2000, 122, 9542-9543, JP2007-531704T and others, such as kinin, quinidine, $(DHQ)_2$PHAL, $(DHQD)_2$PHAL, $(DHQ)_2$PYR, $(DHQD)_2$PYR, $(DHQ)_2$AQN, $(DHQD)_2$AQN, DHQ-CLB, DHQD-CLB, DHQ-MEQ, DHQD-MEQ, DHQ-AQN, DHQD-AQN, DHQ-PHN and DHQD-PHN; a thiourea derivative described in J.Org.Chem., 2008, 73, 2454-2457; a compound represented by the following formulae (5) and (6):

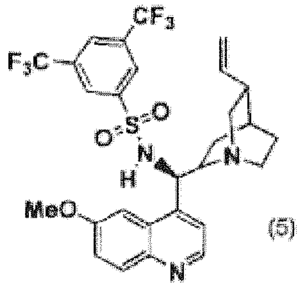

(5)

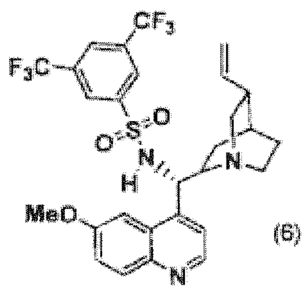

(6)

which is described in Angew.Chem.Int.Ed., 2008, 47, 7822-7875, and derived from quinidine or kinin; and the like.

Among the above examples, the asymmetric organocatalyst is preferably an optically active tertiary amine, and more preferably a compound having an amine unit represented by the following formula (8) :

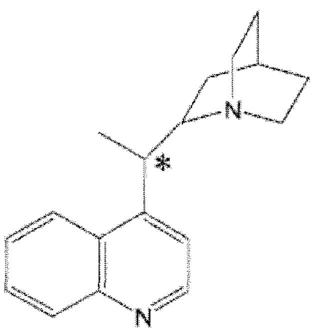

(8)

wherein * indicates an asymmetric carbon atom.

The compound having the amine unit (8) is preferably $(DHQ)_2$AQN, $(DHQD)_2$AQN, the compound (5) or the compound

(6), and more preferably the compound (5) or the compound (6).

The amount of the asymmetric organocatalyst to be used is not particularly limited, and is preferably small in terms of cost. The amount relative to 1 mole of the anhydride (3) is preferably not more than 5 mole, more preferably not more than 1 mole, and particularly preferably not more than 0.2 mole. In addition, the amount of the asymmetric organocatalyst to be used relative to 1 mole of the acid anhydride (3) is exemplified by not less than 0.0001 mole, more preferably not less than 0.001 mole, and particularly preferably not less than 0.1 mole.

The alcohol is exemplified by methanol, ethanol, n-propanol, isopropanol, n-butanol, isobutanol, allyl alcohol, butenol, benzyl alcohol, 1-phenethyl alcohol and the like. The alcohol is methanol, ethanol or benzyl alcohol, more preferably methanol or ethanol, and particularly methanol.

When the amount of the alcohol is too much, stereoselectivity may become low. The amount of the alcohol relative to 1 mole of the acid anhydride (3) is exemplified by not more than 30 mole, preferably not more than 20 mole, and more preferably not more than 10 mole. Also, the amount of the alcohol relative to 1 mole of the acid anhydride (3) is exemplified by not less than 1 mole, preferably not less than 3 mole, and more preferably not less than 5 mole.

It is recommended that the acid anhydride (3) is reacted with an alcohol in a solvent. Such a solvent is not limited as long as the solvent does not negatively affect on the reaction, and specifically exemplified by the organic solvents described in the above "1-2. Means for improving optical purity" of the first route other than an alcohol solvent.

Such a solvent is preferably an ether solvent such as tetrahydrofuran, diethylether, 1,4-dioxane, methyl tert-butyl ether, cyclopentyl methyl ether and ethylene glycol dimethyl ether; an ester solvent such as ethyl acetate, n-propyl acetate and isopropyl acetate; an aromatic hydrocarbon solvent such as benzene and toluene; an aliphatic hydrocarbon solvent such as pentane, hexane, heptane and methylcyclohexane, more preferably tetrahydrofuran, methyl tert-butyl ether, ethyl acetate, isopropyl acetate, toluene or hexane, and particularly preferably tetrahydrofuran or methyl tert-butyl ether.

One of the solvent may be used alone, and two or more solvents may be used in combination. When two or more solvents are used in combination, the mixing ratio is not particularly limited.

The amount of the solvent to be used relative to 1 part by mass of the acid anhydride (3) is preferably not more than 100 parts by mass and more preferably not more than 50 parts by mass, since too much solvent is not preferred in terms of cost and aftertreatment.

It is preferred that the reaction temperature of the step is adjusted to ensure compatibility between stereoselectivity and reaction speed. The reaction temperature is preferably not less than -80°C, more preferably not less than -50°C, and particularly preferably not less than -20°C. In addition, the temperature is preferably not more than 50°C, more preferably not more than 30°C, and particularly preferably not more than 10°C.

The reaction time is not limited, and may be appropriately adjusted. The reaction time is preferably not more than 100 hours, and more preferably not less than 1 hour and not more than 50 hours.

The order of adding the reactants in the reaction is not limited, and it is preferred that an alcohol is added to a solution containing the acid anhydride (3), the asymmetric organocatalyst and the solvent.

After the reaction, a general aftertreatment for obtaining the target compound from a reaction mixture may be carried out. For example, the solvent may be distilled away from the reaction mixture to obtain the target compound by heating under reduced pressure or the like. Alternatively, the target compound can be obtained by adding an acid such as hydrochloric acid and nitric acid to the reaction mixture and then extracting the target compound using an organic solvent. The thus obtained monoester (4) has high purity sufficient for the use in the following step. However, the purity may be further improved with a conventional purification method such as crystallization, fractional distillation, washing by dissolving the monoester (4) in other solvent, and column chromatography.

The monoamide (2) can be produced by carrying out aminolysis reaction of the monoester (4). For example, the aminolysis reaction can be easily carried out by treating the monoester (4) with ammonia water. As ammonia water, commercially available ammonia water of not less than 25 mass% and not more than 30 mass % may be used, or ammonia water having an arbitrary concentration, for example not less than 30 mass%, may be prepared to be used by introducing ammonia gas into water.

The amount of the ammonia to be used relative to 1 mole of the monoester (4) is preferably not less than 1 mole, more preferably not less than 5 mole, and particularly preferably not less than 10 mole. In addition, the amount of ammonia relative to 1 mole of the monoester (4) is preferably not more than 100 mole, more preferably not more than 50 mole, and particularly preferably not more than 30 mole.

The solvent used for the aminolysis reaction is not limited as long as the solvent does not negatively affect on the reaction, and specifically exemplified by water and the organic solvent described in the above "1-2. Means for improving optical purity" of the first route.

The solvent is preferably water; an alcohol solvent such as methanol, ethanol, n-propanol, isopropanol, n-butanol, tert-butanol and ethylene glycol; an ether solvent such as tetrahydrofuran, diethylether, 1,4-dioxane, methyl tert-butyl ether, cyclopentyl methyl ether and ethylene glycol dimethyl ether; an aromatic hydrocarbon solvent such as benzene and toluene; a ketone solvent such as acetone and methyl ethyl ketone; a nitrile solvent such as acetonitrile

and propionitrile, and more preferably water, methanol, ethanol, n-propanol, isopropanol, n-butanol, tetrahydrofuran methyl tert-butyl ether, toluene, acetone or acetonitrile. One of the solvent may be used alone, and two or more solvents may be used in combination. When two or more solvents are used in combination, the mixing ratio is not particularly limited.

The amount of the solvent to be used relative to 1 part by mass of the monoester (4) is preferably not more than 50 parts by mass and more preferably not more than 20 parts by mass, since too much solvent is not preferred in terms of cost and aftertreatment.

The temperature for the aminolysis reaction is exemplified by not less than -30°C and preferably not less than -10°C in terms of reaction speed. In addition, the temperature is exemplified by not more than 100°C and preferably not more than 50°C.

The reaction time is not limited, and may be appropriately adjusted. The reaction time is preferably not more than 100 hours, and more preferably not less than 1 hour and not more than 50 hours.

In the aminolysis step, the order of adding the monoester (4), ammonia water and the solvent is not limited.

After the reaction, a general aftertreatment for obtaining the target compound from a reaction mixture may be carried out. For example, ammonia and the solvent may be distilled away from the reaction mixture by heating under reduced pressure or the like. Alternatively, the reaction mixture may be separated into an organic layer and an aqueous layer to remove the organic layer. After the solvent is distilled away or the organic layer is removed, the monoamide (2) can be precipitated as a solid by adding an acid such as hydrochloric acid and nitric acid to the reaction mixture, and then isolated by filtration.

The thus obtained monoamide (2) has high purity sufficient for the use in the subsequent step. In addition, the yield from the acid anhydride (3) is also high, and is exemplified by not less than 55% and preferably not less than 60%. Furthermore, the optical purity is also sufficiently high, and is exemplified by not less than 90%ee. For further improving the optical purity of the monoamide (2), the method for improving optical purity described in the first route may be used.

For example, the acid anhydride (3) can be produced by dehydrating the compound (9) (dicarboxylic acid (9)). From the monoamide (2) obtained as the above, the compound (10) (aminocarboxilic acid (10)) can be produced as the first route.

3. Third route

In the third route, the monoester (4) is produced by hydrolyzing a 3-substituted glutaric acid diester represented by the following formula (7):

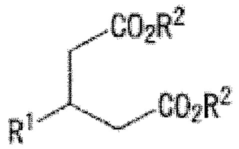

(7)

wherein $R^1$ and $R^2$ are the same as the above,
using an enzyme having an asymmetric hydrolysis activity, and then the monoamide (2) is produced by carrying out an aminolysis reaction of the monoester (4). Hereinafter, the enzyme is referred to as esterase type enzyme. The optical purity of the compound (7) (diester (7)) is generally low, and the compound (7) is typically a racemate.

The esterase type enzyme is not limited as long as the enzyme has the above activity, and lipase is included therein. The lipase is exemplified by α-chymotrypsin, lipase SP526, lipase SP524, Subtilisin A, protease SP539, lipase SP525, lipase D AMANO, CAL-B, Novozyme 435 and the like. The esterase other than lipase is exemplified by esterase derived from hog liver (PLE). The esterase type enzyme is particularly preferably lipase, especially CAL-B and Novozyme 435.
It is preferred that the esterase, preferably lipase, is immobilized.

The amount of the esterase type enzyme relative to 1 part by mass of the diester (7) is exemplified by not more than 10 parts by mass and more preferably not more than 1 part by mass.

As the solvent used in the reaction, water is used.

The amount of water to be used is not limited, and the amount relative to 1 part by mass of the diester (7) is preferably not more than 100 parts by mass, and more preferably not less than 1 part by mass and not more than 10 parts by mass.

In addition, an organic solvent may be added to improve the solubility of the diester (7) as a raw material compound in the ester hydrolysis reaction in order to promote the reaction.

The organic solvent is not particularly limited as long as the solvent does not negatively affect on the reaction, and specifically exemplified by the organic solvents described in the above "1-2. Means for improving optical purity" of the first route.

The solvent is preferably an alcohol solvent such as methanol, ethanol, n-propanol, isopropanol, n-butanol, tert-butanol and ethylene glycol; an ether solvent such as tetrahydrofuran, diethylether, 1,4-dioxane, methyl tert-butyl ether, cyclopentyl methyl ether and ethylene glycol dimethyl ether; an aromatic hydrocarbon solvent such as benzene and toluene; a ketone solvent such as acetone and methyl ethyl ketone; a nitrile solvent such as acetonitrile and propionitrile, and more preferably methanol, ethanol, n-propanol, isopropanol, n-butanol, tetrahydrofuran, methyl tert-butyl ether, toluene, acetone or acetonitrile. One of the solvent may be used alone, and two or more solvents may be used in combination. When two or more solvents are used in combination, the mixing ratio is not particularly limited.

The amount of the organic solvent to be used relative to 1 part by mass of the diester (7) is preferably not more than 50 parts by mass and more preferably not more than 20 parts by mass, since too much solvent is not preferable in terms of cost and aftertreatment.

It is also preferred that the reaction temperature of the ester hydrolysis step is adjusted to ensure compatibility between stereoselectivity and reaction speed. The reaction temperature is preferably not less than 10°C, more preferably not less than 20°C, and more preferably not less than 40°C. In addition, the reaction temperature is preferably not more than 70°C, more preferably not more than 60°C, and even more preferably not more than 55°C.

It is preferred that the pH for the reaction is adjusted to ensure compatibility between stereoselectivity and reaction speed. The pH is preferably not less than 4, more preferably not less than 5, and particularly preferably not less than 6. In addition, the pH is preferably not more than 11, more preferably not more than 10, and particularly preferably not more than 9.

The reaction time is not limited and may be appropriately adjusted. The reaction time is preferably not more than 100 hours, and more preferably not less than 1 hour and not more than 50 hours.

In the ester hydrolysis reaction, the order of adding the diester (7), the esterase type enzyme and the solvent, i.e. water and the organic solvent, is not particularly limited.

After the reaction, a general aftertreatment for obtaining the target compound from a reaction mixture may be carried out. For example, the target compound can be precipitated as a solid by adding an acid such as hydrochloric acid and nitric acid to the reaction mixture for neutralization, and then isolated by filtration. When a processed microorganism, an immobilized enzyme or the like is used as an esterase type enzyme, it is preferred that the processed microorganism, an immobilized enzyme or the like having an esterase activity is separated by filtration from the reaction mixture after the reaction in order to use the processed microorganism or the like in other reaction.

The monoamide (2) can be produced by carrying out an aminolysis reaction of the thus obtained monoester (4) as the second route. The yield from the diester (7) to the monoamide (2) is high, and is exemplified by not less than 55%, preferably not less than 60%, and more preferably not less than 70%. In addition, the optical purity is also sufficiently high, and is exemplified by not less than 90%ee, preferably not less than 93%ee, and more preferably not less than 95%ee. For further improving the optical purity of the monoamide (2), the method for improving optical purity described in the first route may be used.

For example, the diester (7) can be produced by diesterizing the dicarboxylic acid (9). The aminocarboxylic acid (10) can be produced from the thus obtained monoamide (2) as the first route.

## EXAMPLES

Hereinafter, the present invention is described in more detail with Examples; however, the present invention is not restricted by the Examples.

### Reference Example 1: Synthesis of 3-(4-chlorophenyl)glutaric acid diamide

In methanol (60ml), 3- (4-chlorophenyl) glutaric acid (racemate, 10.0 g, 41.2 mmol) was dissolved. To the solution, thionyl chloride (12.3 g, 103 mmol) was added dropwise. After the dropwise addition, the mixture was stirred for 2 hours. Then, the solvent was removed under reduced pressure, to obtain dimethyl 3-(4-chlorophenyl)glutarate (11.1. g, 41.2 mmol) as a white solid.

The above solid of dimethyl 3-(4-chlorophenyl)glutarate (8.0 g, 30.0 mmol) was added into a pressure tight vessel having 200 mL volume. A mixed solution of ammonia/methanol (80 g, 800 mmol) was added thereto, and then the mixture was heated up to 80°C.

The mixture was stirred for 3 days, and then partially concentrated. The precipitated solid was obtained by filtration. The obtained solid was dried under reduced pressure, to obtain 3-(4-chlorophenyl)glutaric acid diamide (6.5 g, 27.0 mmol) as a white solid.

### Reference Example 2: Synthesis of 3-n-propylglutaric acid diamide

In methanol (50 ml), 3-n-propylglutaric acid (racemate. 8.0 g, 45.9 mmol) was dissolved. To the solution, thionyl

chloride (13.7 g, 115 mmol) was added dropwise. After the dropwise addition, the mixture was stirred for 2 hours. Then, the solvent was removed under reduced pressure, to obtain dimethyl 3 -n-propylglutarate (9.3 g, 45.9 mmol) as an oil.

The above oil of dimethyl 3-n-propylglutarate (9. 0 g, 44.5 mmol) was added into a pressure tight vessel having 200 mL volume. A mixed solution of ammonia/methanol (90 g, 900 mmol) was added thereto, and then the mixture was heated up to 80°C.

The mixture was stirred for 5 days, and then partially concentrated. The precipitated solid was obtained by filtration. The obtained solid was dried under reduced pressure, to obtain 3-n-propylglutaric acid diamide (2.3 g, 13.4 mmol) as a solid.

Reference Example 3: Stereoselective hydrolysis of 3-propylglutaric acid diamide

Each microorganism described in Table 1 was inoculated in a sterile nutritive medium (8 ml, glycerol 1.0%, peptone 0.5%, malt extract 0.3%, yeast extract 0.3%, ε-caprolactam 0.1%, pH7.0), and cultured with shaking in a test tube at 30°C for 72 hours. After the culture, the microorganism was collected by centrifugation and dispersed in a 100 mM phosphate buffer (2 ml, pH7.0).

The microorganism dispersion (1 ml) was mixed with the 3-n-propylglutaric acid diamide obtained in Reference Example 2 (2 mg) as a substrate. The mixture was shaken at 30°C for 24 hours, to synthesize 3-n-propylglutaric acid monoamide. After the reaction, a solid was removed by centrifugation. The substrate and the product in the mixture were analyzed with high-performance liquid chromatography, to obtain conversion rate (%).

In addition, the product was treated with phenacyl bromide. The obtained derivative was analyzed with high-performance liquid chromatography, to obtain optical purity (%ee).

The results were shown in Table 1.

Table 1

| Microorganism | Conversion *rate* (%) | Optical *purity* (%ee) | Absolute *configuration* |
|---|---|---|---|
| *Acinetobacter* sp. | 93.6 | 70.2 | R |
| *Acinetobacter* sp. | 76.8 | 76.1 | R |
| *Rhodococcus erythropolis* IFO12539 | 89.5 | 76.8 | R |
| *Rhodococcus erythropolis* IAM1474 | 82.8 | 77.9 | R |
| *Rhodococcus ervthropolis* IAM12122 | 90.6 | 82.0 | R |

The conversion rate and optical purity in Table 1 and Table 2 were calculated by the following formulae from the amount obtained by the following measuring method.

$$\text{Conversion rate (\%)} = [(\text{product amount}) / (\text{substrate amount} + \text{product amount})] \times 100$$

$$\text{Optical purity (\%ee)} = [(A - B) / (A + B)] \times 100$$

whrerein A and B represent the amount of enantiomers, and A > B.

<Condition of high-performance liquid chromatography>

[For conversion rate]
Column: 5C18-ARII (4.6 mmφ x 250 mm, manufactured by Nacalai Tesque)
Eluent in the case of Table 1: 20 mM phosphoric acid aqueous solution (pH 2.5) / acetonitrile = 8 / 2 by mass
Eluent in the case of Table 2: 20 mM phosphoric acid aqueous solution (pH 2.5) / acetonitrile = 7 / 3 by mass
Flow rate: 1.0 ml/min
Column temperature: 30°C
Wavelength for the measurement: 210 nm

[For optical purity]
Column in the case of Table 1: CHIRALPAK AD-RH (4.6 mmφ x 150 mm, manufactured by Daicel Corporation)
Column in the case of Table 2: SUMICHIRAL OA-7000 (4.6 mmφ x 250 mm, manufactured by Sumika Chemical Analysis Service, Ltd.)

Eluent in the case of Table 1: 20 mM phosphoric acid aqueous solution (pH 2.5) / acetonitrile = 1 / 1 by mass
Eluent in the case of Table 2: 20 mM phosphoric acid aqueous solution (pH 2.5) / acetonitrile = 8 / 2 by mass
Flow rate: 0.5 ml/min
Column temperature: room temperature
Wavelength for the measurement: 210 nm

Reference Example 4: Stereoselective hydrolysis of 3-(4-chlorophenyl)glutaric acid diamide

Each microorganism described in Table 2 was inoculated in a sterile nutritive medium (8 ml, glycerol 1.0%, peptone 0.5%, malt extract 0.3%, yeast extract 0.3%, ε-caprolactam 0.1%, pH7.0), and cultured with shaking in a test tube at 30°C for 72 hours. After the culture, the microorganism was collected by centrifugation and dispersed in a 100 mM phosphate buffer (2 ml, pH7.0).

The microorganism dispersion (1 ml) was mixed with the 3-(4-chlorophenyl)glutaric acid diamide obtained in Reference Example 1 (2 mg) as a substrate. The mixture was shaken at 30°C for 24 hours, to synthesize 3-(4-chlorophenyl)glutaric acid monoamide. After the reaction, a solid was removed by centrifugation. The substrate and the product in the mixture were analyzed with high-performance liquid chromatography, to obtain conversion rate (%) and optical purity (%ee).

The results are shown in Table 2.

Table 2

| Microorganism | Conversion rate (%) | Optical purity (%ee) | Absolute configuration |
|---|---|---|---|
| *Delftia acidovorans* NBRC13582 | 96.5 | 99.0 | S |
| *Delftia acidovorans* NBRC14950 | 80.0 | 99.0 | S |
| *Stenotrophomonas* sp. | 86.8 | 99.0 | S |

Example 1: Crystallization of 3-(4-chlorophenyl)glutaric acid monoamide using methanol and water

The (R) -3- (4-chlorophenyl) glutaric acid monoamide obtained in the Reference Example 4 and racemic monoamide were mixed to adjust the optical purity to be 90.5%ee. The monoamide (0.49 g) was dissolved in 30% sodium hydroxide aqueous solution to obtain an aqueous solution (pH 13.4, 5.0 g). Methanol (5.0 g) was added thereto. Then, concentrated hydrochloride was slowly added thereto to adjust the pH to be 2.3. During the addition, a solid of 3-(4-chlorophenyl)glutaric acid monoamide was precipitated. The mixture was left alone for 1 hour, and the precipitated solid was separated by filtration. The solid was dried under reduced pressure to obtain 0.25 g of 3-(4-chlorophenyl)glutaric acid monoamide (yield: 51%, optical purity: 99.6%ee) as a white solid.

Example 2-1: Crystallization of 3-(4-chlorophenyl)glutaric acid monoamide using isopropanol and water

To (R)-3-(4-chlorophenyl)glutaric acid monoamide (0.49 g, optical purity: 90.5%ee), 30% sodium hydroxide aqueous solution was added to obtain an aqueous solution (pH 13.4, 5.0 g). Isopropanol (2.0 g) was added thereto. Then, concentrated hydrochloride was slowly added thereto to adjust the pH to be 1.8. During the addition, a solid of 3-(4-chlorophenyl)glutaric acid monoamide was precipitated. The mixture was left alone for 1 hour, and the precipitated solid was separated by filtration. The solid was dried under reduced pressure to obtain 0.35 g of 3-(4-chlorophenyl) glutaric acid monoamide (yield: 71%, optical purity: 99.5%ee) as a white solid.

Example 2-2: Crystallization of 3-(4-chlorophenyl)glutaric acid monoamide using isopropanol and water

To (R)-3-(4-chlorophenyl)glutaric acid monoamide (0.49 g, optical purity: 90.5%ee), 30% sodium hydroxide aqueous solution was added to obtain an aqueous solution (pH 13.4, 5.0 g). Isopropanol (5.0 g) was added thereto. Then, concentrated hydrochloride was slowly added thereto to adjust the pH to be 2.5. Subsequently, water (20.0 g) was slowly added. During the addition of water, a solid of (R) -3- (4-chlorophenyl) glutaric acid monoamide was precipitated. The mixture was left alone for 1 hour, and the precipitated solid was separated by filtration. The solid was dried under reduced pressure to obtain 0.35 g of (R)-3-(4-chlorophenyl)glutaric acid monoamide (yield: 71%, optical purity: 99.8%ee) as a white solid.

Example 3: Crystallization of 3-(4-chlorophenyl)glutaric acid monoamide using tetrahydrofuran and water

To (R)-3-(4-chlorophenyl)glutaric acid monoamide (0.46 g, optical purity: 90.4%ee), 30% sodium hydroxide aqueous solution was added to obtain an aqueous solution (pH 14.2, 5.0 g). Tetrahydrofuran (1.5 g) was added thereto. Then, concentrated hydrochloride was slowly added thereto to adjust the pH to be 1.6. During the addition, a solid of (R) -3- (4-chlorophenyl)glutaric acid monoamide was precipitated. The mixture was left alone for 1 hour, and the precipitated solid was separated by filtration. The solid was dried under reduced pressure to obtain 0.26 g of (R)-3-(4-chlorophenyl)glutaric acid monoamide (yield: 57%, optical purity: 99.5%ee) as a white solid.

Example 4: Crystallization of 3-(4-chlorophenyl)glutaric acid monoamide using acetonitrile and water

To (R)-3-(4-chlorophenyl)glutaric acid monoamide (0.46 g, optical purity: 90.4%ee), 30% sodium hydroxide aqueous solution was added to obtain an aqueous solution (pH 14.2, 5.0 g). Acetonitrile (1.5 g) was added thereto. Then, concentrated hydrochloride was slowly added thereto to adjust the pH to be 1.5. During the addition, a solid of (R) -3- (4-chlorophenyl) glutaric acid monoamide was precipitated. The mixture was left alone for 1 hour, and the precipitated solid was separated by filtration. The solid was dried under reduced pressure to obtain 0.38 g of (R)-3-(4-chlorophenyl)glutaric acid monoamide (yield: 83%, optical purity: 98.2%ee) as a white solid.

Example 5: Crystallization of 3- (4-chlorophenyl) glutaric acid monoamide using acetone and water

To (R)-3-(4-chlorophenyl)glutaric acid monoamide (0.46 g, optical purity: 90.4%ee), 30% sodium hydroxide aqueous solution was added to obtain an aqueous solution (pH14.2, 5.0 g). Acetone (1.5 g) was added thereto. Then, concentrated hydrochloride was slowly added thereto to adjust the pH to be 1.3. During the addition, a solid of (R)-3-(4-chlorophenyl)glutaric acid monoamide was precipitated. The mixture was left alone for 1 hour, and the precipitated solid was separated by filtration. The solid was dried under reduced pressure to obtain 0.38 g of (R)-3-(4-chlorophenyl)glutaric acid monoamide (yield: 83%, optical purity: 97.6%ee) as a white solid.

Comparative Example 1: Crystallization of 3-(4-chlorophenyl)glutaric acid monoamide using water

To (R)-3-(4-chlorophenyl)glutaric acid monoamide (0.46 g, optical purity: 90.4%ee), 30% sodium hydroxide aqueous solution was added to obtain an aqueous solution (pH 14.2, 5.0 g). Concentrated hydrochloride was slowly added thereto to adjust the pH to be 1.4. During the addition, a solid of (R)-3-(4-chlorophenyl)glutaric acid monoamide was precipitated. The mixture was left alone for 1 hour, and the precipitated solid was separated by filtration. The solid was dried under reduced pressure to obtain 0.41 g of (R)-3-(4-chlorophenyl)glutaric acid monoamide (yield: 89%, optical purity: 91.0%ee) as a white solid.

Example 6: Synthesis of 3-(4-chlorophenyl)glutaric acid monoamide by the second route

Tetrahydrofuran (2 ml) and 3-(4-chlorophenyl)glutaric anhydride (151 mg, 0.668 mmol) were mixed for dissolution, and a catalyst represented by the above formula (5) (40.0 mg, 0.067 mmol) was added thereto. The mixture was cooled down to -20°C. Further, methanol (214 mg, 6.68 mmol) was added. The mixture was stirred for 22 hours. The temperature was heated up to room temperature. Concentrated hydrochloric acid, water (5 ml) and toluene (10 ml) were added thereto, and then the aqueous layer was removed. To the obtained organic layer, 28% ammonia water (1.13 g, 18.6 mmol) was added. The mixture was stirred for 48 hours. After the organic layer was removed, concentrated hydrochloric acid was added thereto to precipitate a solid. The mixture was left alone for 1 hour, and then the precipitated solid was separated by filtration. The solid was dried under reduced pressure to obtain 112.5 mg of (R)-3-(4-chlorophenyl) glutaric acid monoamide (yield: 70%, optical purity: 92.0%ee) as a white solid.

Then, the obtained solid of (R)-3-(4-chlorophenyl)glutaric acid monoamide (80.0 mg) was dissolved in 30% sodium hydroxide aqueous solution (0.80 g). After isopropanol (0.24 g) was added thereto, concentrated hydrochloric acid was slowly added to adjust the pH to be 1.8. During the addition, a solid of (R)-3-(4-chlorophenyl)glutaric acid monoamide was precipitated. The mixture was left alone for 1 hour, and then the precipitated solid was separated by filtration. The solid was dried under reduced pressure to obtain 65.0 mg of (R)-3-(4-chlorophenyl)glutaric acid monoamide (yield: 81%, optical purity: 99.5%ee) as a white solid.

Example 7: Synthesis of 3-(4-chlorophenyl)glutaric acid monoamide by the third route

To 3-(4-chlorophenyl)glutaric acid dimethyl ester (15.0 g, 0.0554mol), water (150 ml) and Novozym 435 as an immobilized enzyme (1.5 g) were added. The mixture was heated up to 50°C. To the mixture, 30% sodium hydroxide

aqueous solution was added to adjust the pH to be 7. Then, the mixture was stirred for 24 hour with maintaining the pH to be 7. After the immobilized enzyme was removed by filtration, concentrated hydrochloric acid was added to adjust the pH to be 2 and extraction was carried out using toluene. To the obtained organic layer, 28% ammonia water (67.3 g, 1.11 mol) was added. The mixture was stirred for 48 hours. After the organic layer was removed, concentrated hydrochloric acid was added. As a result, a solid was precipitated. After the aqueous layer was left alone for 1 hour, the precipitated solid was separated by filtration and dried under reduced pressure, to obtain 9.73 g of (R)-3-(4-chlorophenyl) glutaric acid monoamide (yield: 73%, optical purity: 96.3%ee) as a white solid.

In addition, the above solid of (R)-3-(4-chlorophenyl)glutaric acid monoamide (8.00 g) was dissolved in a sodium hydroxide aqueous solution (80.0 g). Isopropanol (16.0 g) was added and further concentrated hydrochloric acid was slowly added thereto to adjust the pH to be 2.0. During the addition, a solid of (R)-3-(4-chlorophenyl)glutaric acid monoamide was precipitated. After the mixture was left alone for 1 hour, the precipitated solid was separated by filtration and dried under reduced pressure, to obtain 7.03 g of (R)-3-(4-chlorophenyl)glutaric acid monoamide (yield: 88%, optical purity: 99.6%ee) as a white solid.

INDUSTRIAL APPLICABILITY

According to the present invention, a 3-substituted glutaric acid monoamide having high optical purity can be obtained. Such a 3-substituted glutaric acid monoamide is useful as an intermediate for a drug.

## Claims

1. A process for producing an optically active 3-substituted glutaric acid monoamide, comprising the step of precipitating the optically active 3-substituted glutaric acid monoamide by mixing an acid and a mixed liquid containing an optically active 3-substituted glutaric acid monoamide represented by the following formula (2):

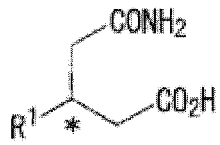

(2)

wherein * indicates an asymmetric carbon atom; $R^1$ is a $C_{1-8}$ alkyl group, a $C_{2-8}$ alkenyl group, a $C_{2-8}$ alkynyl group, a $C_{4-20}$ aryl group or a $C_{5-20}$ aralkyl group; and the alkyl group, the alkenyl group, the alkynyl group, the aryl group and the aralkyl group may have a substituent,
a basic compound, water and an organic solvent.

2. The process according to claim 1, wherein $R^1$ is an n-propyl group or a 4-chlorophenyl group.

3. The process according to claim 1 or 2, wherein the organic solvent is at least one selected from the group consisting of methanol, ethanol, isopropanol, tetrahydrofuran, acetonitrile and acetone.

4. The process according to any one of claims 1 to 3, wherein the amount of the organic solvent is not less than 0.5 parts by mass and not more than 50 parts by mass relative to 1 part by mass of the optically active 3-substituted glutaric acid monoamide.

5. The process according to any one of claims 1 to 4, wherein the basic compound is at least one selected from the group consisting of an alkali metal salt, ammonia and triethylamine.

6. The process according to any one of claims 1 to 5, wherein the pH of the mixed liquid is not less than 6.

7. A process for producing an optically active 3-substituted glutaric acid monoamide, comprising the steps of:

reacting a 3-substituted glutaric anhydride represented by the following formula (3):

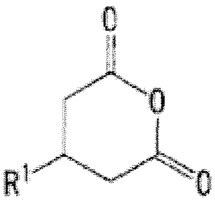 (3)

wherein $R^1$ is a $C_{1-8}$ alkyl group, a $C_{2-8}$ alkenyl group, a $C_{2-8}$ alkynyl group, a $C_{4-20}$ aryl group or a $C_{5-20}$ aralkyl group; and the alkyl group, the alkenyl group, the alkynyl group, the aryl group and the aralkyl group may have a substituent,
with an alcohol in the presence of an asymmetric organocatalyst, to obtain an optically active 3-substituted glutaric acid monoester represented by the following formula (4):

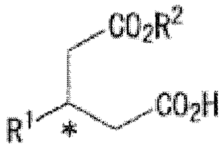 (4)

wherein * indicates an asymmetric carbon atom; $R^1$ is the same as the above; $R^2$ corresponds to a residue obtained by removing the OH group from the alcohol, and is an optionally substituted $C_{1-8}$ alkyl group, an optionally substituted $C_{2-8}$ alkenyl group or an optionally substituted $C_{7-15}$ aralkyl group; and
then carrying out an aminolysis reaction of the optically active 3-substituted glutaric acid monoester (4);
wherein the optically active 3-substituted glutaric acid monoamide is represented by the following formula (2):

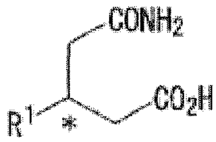 (2)

wherein $R^1$ and * are the same as the above.

**8.** The process according to claim 7, wherein the asymmetric organocatalyst is an optically active tertiary amine compound.

**9.** The process according to claim 7, wherein the asymmetric organocatalyst is a compound represented by the following formula (5) or the following formula (6):

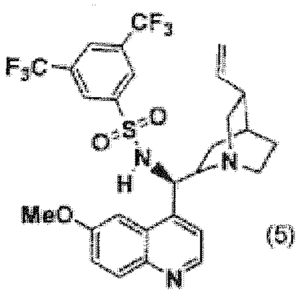 (5) 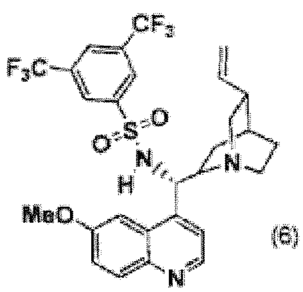 (6)

**10.** The process according to any one of claims 7 to 9, wherein the alcohol is methanol, ethanol or benzyl alcohol.

**11.** A process for producing an optically active 3-substituted glutaric acid monoamide, comprising the steps of:

hydrolyzing a 3-substituted glutaric acid diester represented by the following formula (7):

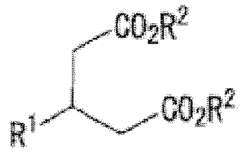

(7)

wherein $R^1$ is a $C_{1-8}$ alkyl group, a $C_{2-8}$ alkenyl group, a $C_{2-8}$ alkynyl group, a $C_{4-20}$ aryl group or a $C_{5-20}$ aralkyl group; the alkyl group, the alkenyl group, the alkynyl group, the aryl group and the aralkyl group may have a substituent; $R^2$ is an optionally substituted $C_{1-8}$ alkyl group, an optionally substituted $C_{2-8}$ alkenyl group or an optionally substituted $C_{7-15}$ aralkyl group,
by using an enzyme having an asymmetric hydrolysis activity, to obtain an optically active 3-substituted glutaric acid monoester represented by the following formula (4):

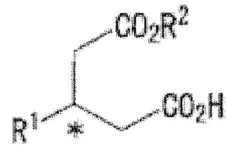

(4)

wherein * indicates an asymmetric carbon atom; and $R^1$ and $R^2$ are the same as the above, and
then carrying out an aminolysis reaction of the optically active 3-substituted glutaric acid monoester (4);
wherein the optically active 3-substituted glutaric acid monoamide is represented by the following formula (2):

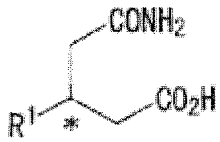

(2)

wherein $R^1$ and * are the same as the above.

**12.** The process according to claim 11, wherein the enzyme having the asymmetric hydrolysis activity is an immobilized lipase.

**13.** The process according to claim 11, wherein the enzyme is Novozyme 435.

**14.** The process according to any one of claims 11 to 13, wherein $R^2$ is a methyl group or an ethyl group.

**15.** A process for producing an optically active 3-substituted glutaric acid monoamide, comprising the steps of:

mixing the optically active 3-substituted glutaric acid monoamide obtained by the process according to any one of claims 7 to 14 with a basic compound, water and an organic solvent to obtain a solution; and
then further mixing the solution with an acid, to precipitate the optically active 3-substituted glutaric acid monoamide.

**INTERNATIONAL SEARCH REPORT**

International application No.
PCT/JP2010/073013

A. CLASSIFICATION OF SUBJECT MATTER
*C07C231/20*(2006.01)i, *C07C231/18*(2006.01)i, *C07C235/84*(2006.01)i, *C12P41/00*(2006.01)i, *C07B53/00*(2006.01)n, *C07B57/00*(2006.01)n, *C07B61/00*(2006.01)n

According to International Patent Classification (IPC) or to both national classification and IPC

B. FIELDS SEARCHED

Minimum documentation searched (classification system followed by classification symbols)
C07C231/20, C07C231/18, C07C235/84, C12P41/00, C07B53/00, C07B57/00, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 11-506098 A (Warner-Lambert Co.), 02 June 1999 (02.06.1999), claims; examples & US 5616793 A & US 5629447 A & EP 828704 A1 & WO 1996/038405 A1 | 1-6,15 |
| A | WO 1997/022578 A1 (FARMARC NEDERLAND B.V.), 26 June 1997 (26.06.1997), claims & US 6051734 A & EP 874804 A1 | 1-6 |

☒ Further documents are listed in the continuation of Box C.　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 March, 2011 (24.03.11) | 05 April, 2011 (05.04.11) |

| Name and mailing address of the ISA/ | Authorized officer |
|---|---|
| Japanese Patent Office | |
| Facsimile No. | Telephone No. |

INTERNATIONAL SEARCH REPORT

International application No.
PCT/JP2010/073013

C (Continuation). DOCUMENTS CONSIDERED TO BE RELEVANT

| Category* | Citation of document, with indication, where appropriate, of the relevant passages | Relevant to claim No. |
|---|---|---|
| A | JP 2008-505980 A (TEVA PHARMACEUTICAL INDUSTRIES LTD.), 28 February 2008 (28.02.2008), claims; examples & US 2006/0276544 A1 & US 2006/0281816 A1 & US 2007/0043241 A1 & US 2007/0073085 A1 & US 2007/0287748 A1 & US 2007/0287859 A1 & US 2007/0287860 A1 & EP 1879851 A1 & EP 1879852 A1 & WO 2006/122255 A1 | 1-6 |
| X<br>Y | US 2007/0293694 A1 (Lilach HEDVATI et al.), 20 December 2007 (20.12.2007), claims; paragraphs [0012] to [0014], [0024] (Family: none) | 7-8<br>9-10 |
| X<br>Y | WO 2007/139933 A2 (TEVA PHARMACEUTICAL INDUSTRIES LTD.), 06 December 2007 (06.12.2007), claims; examples & US 2007/0293694 A1 & EP 2019817 A1 & WO 2007/139933 A2 & CA 2649115 A & KR 10-2008-0027880 A & CN 101448779 A | 7-8<br>9-10 |
| X<br>Y | CN 101514167 A (Meng KUN), 26 August 2009 (26.08.2009), claims; examples (Family: none) | 7-8<br>9-10 |
| Y | Sang Ho OH et al., Angewandte Chemie International Edition, 2008, 47(41), pp.7872-7875 | 9-10 |
| X | Marvin S. Hoekstra et al., Organic Process Research & Development, 1(1), 1997, pp.26-38 | 11-14 |

**INTERNATIONAL SEARCH REPORT**

International application No.
PCT/JP2010/073013

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. ☒ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

**INTERNATIONAL SEARCH REPORT**

International application No.
PCT/JP2010/073013

Continuation of Box No.III of continuation of first sheet(2)

A common matter among the inventions described in claims 1-6, the inventions described in claims 7-10 and the inventions described in claims 11-15 is a compound (2).

However, the compound is disclosed in document 1 shown below, and is therefore not novel and cannot be regarded as a special technical feature in the meaning within PCT Rule 13.2.

Consequently, the inventions described in claims 1-6, the inventions described in claims 7-10 and the inventions described in claims 11-16 cannot be regarded as a group of inventions so linked as to form a single general inventive concept.

Thus, the present application includes the following three groups of inventions.

Group 1: The inventions relating to a method for treating the compound (2) which has had an optical activity already (claims 1-6).
Group 2: The inventions relating to a process for producing the compound (2), which comprises a step of treating a compound (3) with an asymmetric organic catalyst (claims 7-10).
Group 3: The inventions relating to a process for producing the compound (2), which comprises a step of treating a compound (7) with an enzyme having an asymmetric hydrolytic activity (claims 11-15).

Document 1: WO 97/22578 A1 (FARMARC NEDERLAND B.V.) 26 June 1997 (26.06.1997), claims and others.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9722578 A
- WO 9835025 A
- JP 2007119452 A
- JP 2007531704 T


### Non-patent literature cited in the description


- *Journal of the Chemical Society, Perkin Transactions,* 1997, vol. 2 (4), 763-768
- *Tetrahedron Asymmetry,* 2001, vol. 12, 3367-3373
- *Organic Letters,* 2006, vol. 8 (7), 1351-1354
- *J.Am.Chem.Soc.,* 2000, vol. 122, 9542-9543
- *J.Org.Chem.,* 2008, vol. 73, 2454-2457
- *Angew.Chem.Int.Ed.,* 2008, vol. 47, 7822-7875